# EUROPÄISCHE PATENTANMELDUNG

(11) **EP 3 248 962 A1**
(43) Veröffentlichungstag der Anmeldung: **29.11.2017**
(21) Anmeldenummer: 16171095.9
(22) Anmeldetag: 24.05.2016
(51) Int. Cl.: C07C 273/04, C07C 2/84, C07C 11/02, C07C 11/04

(54) **PROZESS UND ANLAGENVERBUND ZUR HERSTELLUNG VON HARNSTOFF**

(71) Anmelder: Linde Aktiengesellschaft, 80331 München (DE)
(72) Erfinder: Zimmermann, Heinz, 81476 München (DE); Fritz, Helmut, 81375 München (DE); Haidegger, Ernst, 85521 Riemerling (DE); Kemper, Rainer, 81375 München (DE)
(74) Vertreter: m patent group

(57) **Zusammenfassung**

Die Erfindung betrifft einen Prozess (100), bei dem unter Einbeziehung eines Luftzerlegungsverfahrens (10) ein sauerstoffreicher Stoffstrom (b) gebildet wird, der mit einem methanreichen Stoffstrom (e) einem Verfahren zur oxidativen Methankopplung (20) unterworfen wird. Aus einem Produktstrom (e) des Verfahrens zur oxidativen Methankopplung (20) wird ein kohlendioxidreicher Stoffstrom (i) gebildetund einem Harnstoffsyntheseverfahren (50) unterworfen. Ein entsprechender Anlagenverbund ist ebenfalls Gegenstand der Erfindung.

## Beschreibung

Die Erfindung betrifft einen Prozess zur Herstellung von Harnstoff und einen entsprechenden Anlagenverbund gemäß den Oberbegriffen der jeweiligen unabhängigen Patentansprüche.

### Stand der Technik

Methan, beispielsweise aus Erdgas, wird derzeit überwiegend verfeuert. Eine alternative stoffliche Nutzung ist jedoch aus wirtschaftlicher Sicht von großem Interesse. So befinden sich derzeit Verfahren zur Herstellung höherer Kohlenwasserstoffe aus Methan durch oxidative Methankopplung (engl. Oxidative Coupling of Methane, OCM) in intensiver Entwicklung. Bei der oxidativen Methankopplung handelt es sich um die direkte Umsetzung von Methan in einem oxidativen, heterogen katalysierten Verfahren zu höheren Kohlenwasserstoffen. Entsprechende Verfahren dienen insbesondere zur Herstellung von Ethylen.

Bezüglich weiterer Details der oxidativen Methankopplung sei auf einschlägige Fachliteratur, beispielsweise Zavyalova, U. et al.: Statistical Analysis of Past Catalytic Data on Oxidative Methane Coupling for New Insights into the Composition of High-Performance Catalysts, ChemCatChem 3, 2011, 1935-1947, verwiesen.

Bei der oxidativen Methankopplung wird ein methanreicher Stoffstrom, beispielsweise Erdgas oder ein aus Erdgas gebildeter Stoffstrom, zusammen mit einem sauerstoffreichen Stoffstrom einem Reaktor zugeführt. Es wird ein Produktstrom gebildet, der neben Reaktionsprodukten der oxidativen Methankopplung, insbesondere Ethylen, ggf. Propylen, Wasserstoff, Kohlendioxid, nicht umgesetztes Methan und nicht umgesetzten Sauerstoff enthält. Wird beispielsweise stickstoffhaltiges Erdgas eingesetzt, enthält der Produktstrom auch Stickstoff.

Der für die oxidative Methankopplung eingesetzte sauerstoffreiche Stoffstrom wird typischerweise durch ein Luftzerlegungsverfahren geliefert. Die Herstellung von Luftprodukten mittels entsprechender Luftzerlegungsverfahren ist seit langem bekannt und beispielsweise bei H.-W. Häring (Hrsg.), Industrial Gases Processing, Wiley-VCH, 2006, insbesondere Abschnitt 2.2.5, "Cryogenic Rectification", beschrieben. Die vorliegende Erfindung betrifft dabei insbesondere solche Luftzerlegungsverfahren, die zur Erzeugung gasförmiger, sauerstoffreicher Stoffströme eingerichtet sind.

Es besteht grundsätzlich der Bedarf, Produkte der oxidativen Methankopplung besser zu nutzen und die Gesamtausbeute entsprechender Prozesse zu erhöhen.

### Offenbarung der Erfindung

Diese Aufgabe wird durch einen Prozess zur Herstellung von Harnstoff und einen entsprechenden Anlagenverbund mit den Merkmalen der unabhängigen Patentansprüche gelöst. Ausgestaltungen sind jeweils Gegenstand der abhängigen Patentansprüche sowie der nachfolgenden Beschreibung.

Flüssige und gasförmige Stoffströme können im hier verwendeten Sprachgebrauch reich oder arm an einer oder mehreren Komponenten sein, wobei der Begriff "reich" für einen Gehalt von wenigstens 50%, 75%, 90%, 95%, 99%, 99,5%, 99,9% oder 99,99% und der Begriff "arm" für einen Gehalt von höchstens 50%, 25%, 10%, 5%, 1%, 0,1% oder 0,01% auf molarer, Gewichts- oder Volumenbasis stehen kann.

Wird ein Stoffstrom im hier verwendeten Sprachgebrauch "unter Einbeziehung" eines bestimmten Verfahrens, beispielsweise eines Luftzerlegungsverfahrens oder eines Verfahrens zur oxidativen Methankopplung, gebildet, schließt dies explizit nicht aus, dass auch weitere Verfahren, insbesondere Trennverfahren, an der Bildung des Stoffstroms beteiligt sein können. Ebenfalls schließt die Formulierung nicht aus, dass durch entsprechende Verfahren jeweils auch zusätzliche Stoffströme gleicher oder abweichender Zusammensetzung gebildet werden können.

### Vorteile der Erfindung

Vor dem oben erläuterten Hintergrund schlägt die vorliegende Erfindung einen Prozess vor, bei dem unter Einbeziehung eines Luftzerlegungsverfahrens ein sauerstoffreicher Stoffstrom gebildet wird, der mit einem methanreichen Stoffstrom einem Verfahren zur oxidativen Methankopplung unterworfen wird. Insoweit unterscheidet sich der erfindungsgemäße Prozess nicht von den eingangs erläuterten Prozessen des Standes der Technik. Die Erfindung schlägt nun aber zusätzlich vor, in einem derartigen Prozess aus einem Produktstrom des Verfahrens zur oxidativen Methankopplung einen kohlendioxidreichen Stoffstrom zu bilden, der einem Syntheseverfahren zur Erzeugung von Harnstoff unterworfen wird.

Wie sich im Rahmen der vorliegenden Erfindung herausgestellt hat, eignet sich die erfindungsgemäß vorgeschlagene Kopplung der oxidativen Methankopplung mit einem entsprechenden Syntheseverfahren in besonderer Weise zur Erhöhung der Gesamtwirtschaftlichkeit entsprechender Prozesse.

In einem Produktstrom eines Verfahrens zur oxidativen Methankopplung ist Kohlendioxid in typischerweise nicht unbeträchtlicher Menge enthalten. Bei dem Kohlendioxid handelt es sich bei herkömmlicher Betrachtungsweise um ein unerwünschtes Nebenprodukt. Wie beispielsweise bei Zavyalova et al. (siehe oben) erläutert, tritt bei der oxidativen Kopplung von Methan auch eine nichtselektive Oxidation des Methans und der gebildeten Kohlenwasserstoffe zu Kohlenmonoxid und Kohlendioxid auf. Herkömmlicherweise sollten daher geeignete Katalysatoren für die oxidative Kopplung von Methan nicht nur die Bildung von Methylradikalen katalysieren, die anschließend zu Ethan und Ethylen reagieren, sondern auch die nichtselektive Oxidation des Methans und der gebildeten Kohlenwasserstoffe unterdrücken. Wird ein erfindungsgemäßes Verfahren genutzt, kann Kohlendioxid in vollem Umfang zu Produkten umgesetzt werden, so dass dieser Aspekt geringere Bedeutung hat und größere Freiheiten in der Optimierung eines entsprechenden Katalysators bestehen.

Entsprechendes Kohlendioxid wird typischerweise stromauf eines Trennverfahrens aus entsprechenden Produktströmen entfernt, um ein Ausfrieren und damit eine Verlegung von Anlagenteilen bei den verwendeten Trenntemperaturen und -drücken zu verhindern. Zur Abtrennung von Kohlendioxid kommt typischerweise eine an sich bekannte Kohlendioxidwäsche zum Einsatz. Das dabei gewonnene Kohlendioxid eignet sich insbesondere zur Verwendung in einem Harnstoffsyntheseverfahren, wie es erfindungsgemäß eingesetzt wird. Zur Herstellung des in einem derartigen Harnstoffsyntheseverfahren benötigten Ammoniaks können auch eine Reihe weiterer, in einem entsprechenden Prozess anfallender Verbindungen genutzt werden.

In diesem Zusammenhang ist es insbesondere von Vorteil, wenn zunächst ein Ammoniaksyntheseverfahren durchgeführt und dies ebenfalls in den erfindungsgemäßen Prozess integriert wird. Ammoniak, der in einem entsprechenden Ammoniaksyntheseverfahren gebildet wird, lässt sich anschließend mit dem Kohlendioxid aus dem Produktstrom des Verfahrens zur oxidativen Methankopplung zu Harnstoff umsetzen, wie erfindungsgemäß vorgesehen.

Der Prozess erlaubt in der vorgeschlagenen Prozessvariante beispielsweise eine weitere Integration von Luftzerlegung und oxidativer Methankopplung, indem in der Luftzerlegung gebildeter Stickstoff, der in der oxidativen Methankopplung nicht genutzt wird, dem Ammoniaksyntheseverfahren unterworfen wird. Stickstoff für das Ammoniaksyntheseverfahren kann jedoch auch vollständig oder teilweise aus dem Produktstrom des Verfahrens zur oxidativen Methankopplung stammen.

In dem Ammoniaksyntheseverfahren erforderlicher Wasserstoff kann ebenfalls aus dem Produktstrom des Verfahrens zur oxidativen Methankopplung und/oder aus einem anderen Verfahren bzw. einer anderen Quelle stammen. Beispielsweise kann Methan oder Erdgas, das auch für das Verfahren zur oxidativen Methankopplung zur Verfügung gestellt wird, parallell einem Wasserstoffsyntheseverfahren bekannter Art, beispielsweise einer Dampfreformierung, unterworfen werden. Auf diese Weise gebildeter Wasserstoff kann alleine oder zusammen mit Wasserstoff, der in dem Produktstrom des Verfahrens zur oxidativen Methankopplung enthalten ist, eingesetzt werden. Auf diese Weise kann beispielsweise ein nicht ausreichender oder schwankender Wasserstoffgehalt in dem Produktstrom des Verfahrens zur oxidativen Methankopplung ausgeglichen werden. Die bevorzugte Quelle für Wasserstoff richtet sich auch nach dessen Zugänglichkeit. Erweist sich beispielsweise eine Gewinnung von Wasserstoff aus dem Produktstrom des Verfahrens zur oxidativen Methankopplung als zu aufwendig, kann auch ausschließlich auf Wasserstoff zurückgegriffen werden, der mittels eines separaten Wasserstoffsyntheseverfahrens der erläuterten Art gewonnen wird.
Der Prozess kann also gemäß der soeben erläuterten vorteilhaften Ausführungsform einen unter Einbeziehung des Luftzerlegungsverfahrens gebildeten stickstoffreichen Stoffstrom nutzen. Alternativ oder zusätzlich dazu ist es möglich, Stickstoff, der in dem Produktstrom des Verfahrens zur oxidativen Methankopplung enthalten ist, zu nutzen. Auch aus diesem Stickstoff kann dabei zunächst zusammen mit Wasserstoff Ammoniak synthetisiert werden, der anschließend erfindungsgemäß zusammen mit dem kohlendioxidreichen Stoffstrom dem Syntheseverfahren zur Erzeugung von Harnstoff unterworfen wird. Wird ein unter Einbeziehung des Luftzerlegungsverfahrens gebildeter stickstoffreicher Stoffstrom zur Erzeugung des Ammoniaks genutzt, können beliebige Mengen an Stickstoff bereitgestellt werden, so dass der Prozess völlig unabhängig von ggf. nur in geringen Anteilen oder gar nicht in dem Abstrom des Verfahrens zur oxidativen Methankopplung enthaltenem Stickstoff ist. Eine entsprechende Prozessvariante eignet sich also insbesondere für Fälle, in denen ein Produktstrom des Verfahrens zur oxidativen Methankopplung keinen oder keinen ausreichenden Stickstoffgehalt aufweist, oder zum Ausgleich von Schwankungen in dessen Stickstoffgehalt.

Zwar liegen Temperaturen und Drücke, wie sie in Syntheseverfahren zur Erzeugung von Ammoniakeingesetzt werden, teilweise deutlich über jenen, wie sie in der oxidativen Methankopplung eingesetzt werden. Dennoch entfaltet der Prozess gemäß der soeben erläuterten Ausführungsform der Erfindung besondere Vorteile, wenn in einem entsprechenden Syntheseverfahren zur Erzeugung von Ammoniak der Stickstoff, der in dem Produktstrom des Verfahrens zur oxidativen Methankopplung enthalten ist, genutzt wird. In diesem Fall müssen zumindest keine Verdichtung ausgehend von Atmosphärendruck und/oder keine Temperaturerhöhung ausgehend von Umgebungstemperatur oder ggf. darunter erfolgen, wie sie bei der Verwendung von Stickstoff aus dem Luftzerlegungsverfahren erforderlich sein kann. Die aufzuwendende Energie wird damit deutlich geringer.

Syntheseverfahren zur Erzeugung von Ammoniak und Harnstoff sind grundsätzlich bekannt. Zu beiden Verfahren sei auf Fachliteratur, beispielsweise den Artikel "Ammonia" in Ullmann's Encyclopedia of Industrial Chemistry, Onlineveröffentlichung 15. Dezember 2006, doi:10.1002/14356007.a02_143.pub2, und den Artikel "Urea" in Ullmann's Encyclopedia of Industrial Chemistry, Onlineveröffentlichung 15. Juni 2000, doi:10.1002/14356007.a27_333.pub2, verwiesen.
Wie bereits erwähnt, kann der in dem Produktstrom des Verfahrens zu oxidativen Methankopplung enthaltene Wasserstoff einem Ammoniaksyntheseverfahren unterworfen werden. Die vorliegende Erfindung ermöglicht damit eine vorteilhafte Nutzung des in der oxidativen Methankopplung gebildeten Wasserstoffs.

Insbesondere entfaltet diese Prozessvariante besondere Vorteile in Fällen, in denen der Produktstrom des Verfahrens zu oxidativen Methankopplung Stickstoff enthält, weil dann dieser Stickstoff nicht von dem Wasserstoff getrennt werden muss. Ein entsprechender Produktstrom kann aus unterschiedlichen Gründen Stickstoff enthalten, wobei sich der erfindungsgemäße Prozess in sämtlichen Fällen eignet.

So erweist sich der vorliegende Prozess insbesondere für Fälle als vorteilhaft, in denen der methanreiche Stoffstrom, der dem Verfahren zur oxidativen Methankopplung unterworfen wird, nicht nur geringfügige Mengen an Stickstoff aufweist. Da dieser Stickstoff typischerweise in den Verfahren zur oxidativen Methankopplung nicht oder kaum umgesetzt wird, geht er in den Produktstrom über und muss herkömmlicherweise aufwendig abgetrennt werden. Mit einem Siedepunkt von -196 °C (Stickstoff) und -252 °C (Wasserstoff) stellen Stickstoff und Wasserstoff die Komponenten mit den niedrigsten Siedepunkten in entsprechenden Produktströmen dar. Die übrigen, in nennenswerter Menge in entsprechenden Produktströmen enthaltenen Verbindungen sieden deutlich höher. Eine Trennung von Wasserstoff und Stickstoff würde daher beispielsweise eine aufwändige Tieftemperaturzerlegung oder ein Membranverfahren erfordern, was aus wirtschaftlicher Sicht nachteilig ist und/oder aufwändige zusätzliche Trenneinrichtungen erfordern würde. Entsprechendes gilt auch für eine Abreicherung von Erdgas an Stickstoff, die in vorgelagerten Verfahrensschritten erfolgen müsste.

Wird ein aus einem entsprechenden Produktstrom gebildeter wasserstoffreicher Stoffstrom jedoch einem Ammoniaksyntheseverfahren unterworfen, stört enthaltener Stickstoff hier nicht. Wenn dabei die in dem Produktstrom des Verfahrens zur oxidativen Methankopplung enthaltene Stickstoffmenge zur stöchiometrischen Umsetzung mit Wasserstoff nicht ausreicht, kann im Rahmen der soeben erläuterten Ausführungsform der Erfindung ein weiterer stickstoffreicher Stoffstrom eingesetzt werden, der, wie zuvor erläutert, unter Einbeziehung des Luftzerlegungsverfahrens gebildet werden kann.

Werden stickstoffhaltige, methanreiche Stoffströme als Einsätze für die oxidative Methankopplung eingesetzt, können diese beispielsweise einen Stickstoffgehalt von bis zu 20 Molprozent, insbesondere von 1 bis 5 oder 5 bis 10 Molprozent, aufweisen, wobei der in dem methanreichen Stoffstrom enthaltene Stickstoff teilweise oder vollständig in den wasserstoffreichen Stoffstrom übergeht und in diesem den Ammoniaksyntheseverfahren unterworfen wird. Wie zuvor erläutert, ermöglicht die vorliegende Erfindung in dieser Prozessvariante den Verzicht auf eine Stickstoffabreicherung des Erdgases und/oder eine Trennung von Wasserstoff und Stickstoff in einem entsprechenden Produktstrom.

Die vorliegende Erfindung ermöglicht es in der erläuterten Ausführungsform jedoch auch, energieeffizientere Luftzerlegungsverfahren einzusetzen, weil dem Verfahren zur oxidativen Methankopplung nicht notwendigerweise reiner Sauerstoff in Form des sauerstoffreichen Stoffstroms zugeführt werden muss. Es kann also eine unschärfere Trennung von Stickstoff und Sauerstoff erfolgen. Wie erläutert, wird Stickstoff in entsprechenden Verfahren zur oxidativen Methankopplung nicht oder nur kaum umgesetzt, so dass dieser in einen entsprechenden Produktstrom übergeht. Der in dem Produktstrom enthaltene Stickstoff kann anschließend in einem Ammoniaksyntheseverfahren genutzt werden, wie erläutert. Daher ermöglicht die vorliegende Erfindung auch den Einsatz von sauerstoffreichen Stoffströmen mit einem Gehalt von beispielsweise bis zu 20 Molprozent, insbesondere von 1 bis 5 oder 5 bis 10 Molprozent Stickstoff. Die Erfindung ermöglicht beispielsweise den Einsatz von Luftzerlegungsanlagen mit Mischsäulen. Entsprechende Anlagen und Verfahren sind vielfach an anderer Stelle offenbart, beispielsweise in der EP 1 139 046 B1. Für Details zur Optimierung von Luftzerlegungsanlagen sei auf einschlägige Fachliteratur verwiesen, beispielsweise Kapitel 3.8 in Kerry, F.G., Industrial Gas Handbook: Gas Separation and Purification, Boca Raton: CRC Press, 2006.

Die vorliegende Erfindung ermöglicht jedoch eine noch weitergehende Integration der genannten Prozesskomponenten bzw. Verfahren. So ist es insbesondere auch von Vorteil, wenn aus dem Produktstrom des Verfahrens zur oxidativen Methankopplung ferner ein oder mehrere olefinreiche Stoffströme und unter Einbeziehung des Luftzerlegungsverfahrens ein oder mehrere weiterer sauerstoffreiche Stoffströme gebildet werden. Der oder die olefinreichen Stoffströme und der oder die weiteren sauerstoffreichen Stoffströme können miteinander einem Epoxidierungsverfahren unterworfen werden. Entsprechende Epoxidierungsverfahren können separat für einen olefinreichen Stoffstrom und/oder für mehrere gemeinsam bereitgestellt werden. Es kann insbesondere auch nur ein olefinreicher Stoffstrom epoxidiert werden. Ein oder mehrere entsprechender olefinreicher Stoffströme sind reich an Ethylen und/oder Propylen. Bei einer entsprechenden Epoxidierung wird aus Propylen Propylenoxid und aus Ethylen Ethylenoxid gebildet, also Verbindungen, die sich als Ausgangskomponenten für weitere Reaktionen besonders eignen. Insbesondere kann vorgesehen sein, aus in dem Epoxidierungsverfahren gebildetem Ethylenoxid und/oder Propylenoxid Ethylenglycol und/oder Propylenglycol zu synthetisieren.

Die vorliegende Erfindung ermöglicht auch die Rückführung von Stoffströmen, beispielsweise indem aus dem Produktstrom des Verfahrens zur oxidativen Methankopplung wenigstens ein weiterer Stoffstrom gebildet wird, der erneut dem Verfahren zur oxidativen Methankopplung unterworfen wird. Der wenigstens eine weitere Stoffstrom, der insbesondere Methan enthalten kann, ist dabei vorteilhafterweise arm an oder frei von Stickstoff, er kann jedoch Stickstoff enthalten, wenn im Rahmen des erfindungsgemäßen Verfahrens kontinuierlich Stickstoff aus einem entsprechenden Kreislauf ausgeführt, d. h. beispielsweise dem Ammoniaksyntheseverfahren zugeführt wird.

Die vorliegende Erfindung betrifft ferner einen Anlagenverbund, der eine Luftzerlegungsanlage und wenigstens einen zur Durchführung eines Verfahrens zur oxidativen Methankopplung eingerichteten Reaktor umfasst. Der Anlagenkomplex weist Mittel auf, die dafür eingerichtet sind, unter Einbeziehung eines in der Luftzerlegungsanlage durchgeführten Luftzerlegungsverfahrens einen sauerstoffreichen Stoffstrom zu bilden und diesen mit einem methanreichen Stoffstrom in dem wenigstens einen Reaktor einem Verfahren zur oxidativen Methankopplung zu unterwerfen. Erfindungsgemäß sind Mittel vorgesehen, die dafür eingerichtet sind, aus einem Produktstrom des Verfahrens zur oxidativen Methankopplung einen kohlendioxidreichen Stoffstrom zu bilden und diesen in einem oder mehreren weiteren Reaktoren einem Harnstoffsyntheseverfahren zu unterwerfen.

Ein entsprechender Anlagenverbund ist vorteilhafterweise zur Durchführung eines Verfahrens eingerichtet, wie es zuvor erläutert wurde, und weist hierfür entsprechende Mittel auf. Zu Merkmalen und Vorteilen eines entsprechenden Anlagenkomplexes sei daher auf die obigen Erläuterungen ausdrücklich verwiesen.

Die Erfindung wird nachfolgend unter Bezugnahme auf die beigefügte Zeichnung näher erläutert, die eine bevorzugte Ausführungsform der Erfindung zeigt.

### Kurze Beschreibung der Zeichnung

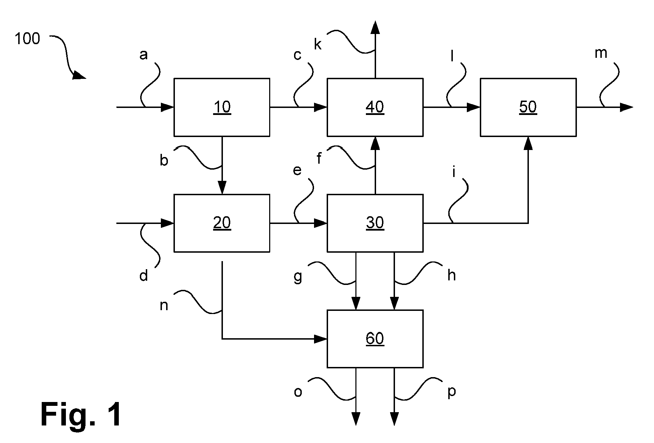

Figur 1 zeigt einen Prozess zur Herstellung von Reaktionsprodukten gemäß einer besonders bevorzugten Ausführungsform der Erfindung.

### Ausführliche Beschreibung der Zeichnung

In Figur 1 ist ein Prozess gemäß einer besonders bevorzugten Ausführungsform der Erfindung in Form eines schematischen Prozessflussdiagramms dargestellt und insgesamt mit 100 bezeichnet.

Der Prozess 100 umfasst ein Luftzerlegungsverfahren 10 und ein Verfahren zur oxidativen Methankopplung 20. Dem Luftzerlegungsverfahren 10 wird Einsatzluft in Form eines Stoffstroms a zugeführt. Im Rahmen des Prozesses 100 einsetzbare Luftzerlegungsverfahren 10 sind vielfach an anderer Stelle beschrieben.

Unter Einsatz eines entsprechenden Luftzerlegungsverfahrens 10 werden im dargestellten Beispiel ein sauerstoffreicher Stoffstrom b und ein stickstoffreicher Stoffstrom c bereitgestellt. Unter Einsatz des Luftzerlegungsverfahrens 10 können jedoch auch beliebige weitere Stoffströme, die Luftzerlegungsprodukte umfassen können, bereitgestellt werden, beispielsweise weitere sauerstoffreiche und/oder stickstoffreiche Stoffströme und/oder Stoffströme, die reich an einem oder mehreren Edelgasen sind, wie grundsätzlich bekannt.

Dem Verfahren zur oxidativen Methankopplung 20 werden im dargestellten Beispiel der sauerstoffreiche Stoffstrom b sowie ein methanreicher Stoffstrom d, bei dem es sich beispielsweise um aufbereitetes oder nicht aufbereitetes Erdgas handeln kann, zugeführt. In dem Verfahren zur oxidativen Methankopplung 20 wird ein Produktstrom e erzeugt, der unter anderem nicht umgesetztes Methan des Stoffstroms d, nicht umgesetzten Sauerstoff des Stoffstroms b, gegebenenfalls in dem Stoffstrom d enthaltene Inertgase wie Stickstoff sowie Reaktionsprodukte der oxidativen Methankopplung wie Wasserstoff, Kohlendioxid, Ethylen oder Propylen enthalten kann.

Der Produktstrom e wird einem Trennverfahren 30, welches nicht kryogene sowie kryogene Trennschritte umfassen kann, unterworfen. Insbesondere kann das Trennverfahren 30 auch eine Gaswäsche umfassen. Unter Einsatz des Trennverfahrens 30 können insbesondere ein wasserstoffreicher Stoffstrom f, ein ethylenreicher Stoffstrom g, ein propylenreicher Stoffstrom h und ein kohlendioxidreicher Stoffstrom i bereitgestellt werden. Der wasserstoffreiche Stoffstrom f, der propylenreiche Stoffstrom g sowie der ethylenreiche Stoffstrom h werden typischerweise in einen oder mehreren kryogenen Trennschritten des Trennverfahrens 30 erzeugt, der kohlendioxidreiche Stoffstrom i wird typischerweise vorab abgetrennt. Im Trennverfahren 30 bzw. in entsprechenden Trennschritten können auch weitere Stoffströme bereitgestellt werden, die jedoch der Übersichtlichkeit halber in Figur 1 nicht veranschaulicht sind.

Im Rahmen des Prozesses 100 in der in Figur 1 veranschaulichten Ausführungsform erfolgt die Durchführung eines Ammoniaksyntheseverfahrens 40, dem im dargestellten Beispiel der stickstoffreiche Stoffstrom c, der unter Einsatz des Luftzerlegungsverfahrens 10 bereitgestellt wird, sowie der wasserstoffreiche Stoffstrom f, der unter Einsatz des Verfahrens zur oxidativen Methankopplung und des nachgeschalteten Trennverfahrens 30 bereitgestellt wird, zugeführt werden. Wie mehrfach erwähnt, kann ein entsprechender wasserstoffreicher Stoffstrom f jedoch auch aus anderen Quellen, beispielsweise einem Dampfreformierungsverfahren, stammen. Grundsätzlich kann Ammoniak ebenfalls aus anderen Quellen stammen.

Es sei betont, dass unter Einsatz des Verfahrens zur oxidativen Methankopplung 20 bzw. des nachgeschalteten Trennverfahrens 30 auch weitere wasserstoffreiche Ströme bereitgestellt werden können, die nicht notwendigerweise vollständig dem Ammoniaksyntheseverfahren 40 zugeführt werden müssen. Ebenso muss der dem Ammoniaksyntheseverfahren 40 zugeführte Stickstoff nicht oder nicht ausschließlich aus dem stickstoffreichen Stoffstrom c aus dem Luftzerlegungsverfahren 10 stammen. Zumindest ein Teil des Stickstoffs kann auch in dem wasserstoffreichen Stoffstrom f enthalten sein, wie oben erläutert, insbesondere wenn dieser aus einem Verfahren zur oxidativen Kopplung von Methan stammt.

Unter Einsatz des Ammoniaksyntheseverfahrens 40 werden im dargestellten Beispiel zwei ammoniakreiche Ströme k und l bereitgestellt. Die in Figur 1 dargestellte besonders bevorzugte Ausführungsform des Prozesses 100 umfasst ein Harnstoffsyntheseverfahren 50. Dem Harnstoffsyntheseverfahren 50 werden dabei der ammoniakreiche Strom I, der unter Einsatz des Ammoniaksyntheseverfahren 40 bereitgestellt wird, und der kohlendioxidreiche Strom i, der unter Einsatz des Verfahrens zur oxidativen Methankopplung 20 und des nachgeschalteten Trennverfahrens 30 bereitgestellt wird, zugeführt. Es versteht sich, dass dem Harnstoffsyntheseverfahren 50 nicht der gesamte, in dem Ammoniaksyntheseschritt 40 gebildete Ammoniak und/oder nicht das gesamte, in dem Verfahren zur oxidativen Methankopplung 20 und dem nachgeschalteten Trennverfahren 30 bereitgestellte Kohlendioxid zugeführt werden müssen. Es können jeweils auch nur Teilmengen der genannten Verbindungen verwendet werden, der Rest kann beispielsweise als Produkt bzw. Nebenprodukt aus einem entsprechenden Prozess 100 ausgeführt werden. Entsprechendes ist in Figur 1 mit den ammoniakreichen Stoffströmen k und I veranschaulicht.

Der ammoniakreiche Stoffstrom k wird im dargestellten Beispiel aus dem Prozess ausgeführt. Unter Einsatz des Harnstoffsyntheseverfahrens 50 wird in der in Figur 1 dargestellten besonders bevorzugten Ausführungsform der Erfindung ein harnstoffreicher Stoffstrom m bereitgestellt und bei Bedarf geeigneten Aufbereitungsschritten zugeführt.

Auch die nachfolgend erläuterten Verfahren sind nicht notwendigerweise Bestandteil eines entsprechenden Prozesses 100. Dies bedeutet, dass der propylenreiche Stoffstrom g und/oder der ethylenreiche Stoffstrom h jeweils auch als Produkte aus einem entsprechenden Prozess 100 ausgeführt werden können.

Im dargestellten Beispiel ist ein Epoxidierungsverfahren 60 veranschaulicht, das auch getrennt für den propylenreichen Stoffstrom g und den ethylenreichen Stoffstrom h oder nur für einen dieser Stoffströme vorgesehen sein kann. Dem Epoxidierungsverfahren 60 wird ferner ein sauerstoffreicher Stoffstrom n zugeführt, der insbesondere unter Einsatz des Luftzerlegungsverfahrens 10 bereitgestellt werden kann. Unter Einsatz des Epoxidierungsverfahrens 60 können ein propylenoxidreicher Stoffstrom o und/oder ein ethylenoxidreicher Stoffstrom p bereitgestellt werden. Auch hier muss dem Epoxidierungsverfahren 60 nicht das gesamte, in dem Verfahren zur oxidativen Methankopplung 20 bzw. dem nachgeschalteten Trennverfahren 30 bereitgestellte Propylen und/oder Ethylen unterworfen werden. Insbesondere können auch Teilströme entsprechenden Propylens bzw. Ethylens als Produkte aus dem Prozess 100 ausgeführt werden.

## Patentansprüche

1. Prozess (100), bei dem unter Einbeziehung eines Luftzerlegungsverfahrens (10) ein sauerstoffreicher Stoffstrom (b) gebildet wird, der mit einem methanreichen Stoffstrom (e) einem Verfahren zur oxidativen Methankopplung (20) unterworfen wird, **dadurch gekennzeichnet, dass** aus einem Produktstrom (e) des Verfahrens zur oxidativen Methankopplung (20) ein kohlendioxidreicher Stoffstrom (i) gebildet und einem Harnstoffsyntheseverfahren (50) unterworfen wird.

2. Prozess (100) nach Anspruch 1, bei dem unter Einbeziehung des Luftzerlegungsverfahrens (10) ferner ein stickstoffreicher Stoffstrom (b) gebildetund einem Ammoniaksyntheseverfahren (40) unterworfen wird.

3. Prozess (100) nach Anspruch 2, bei dem der aus dem Produktstrom (e) ferner ein wasserstoffreicher Stoffstrom (f) gebildet und dem Ammoniaksyntheseverfahren (40) unterworfen wird.

4. Prozess (100) nach Anspruch 3, bei dem der methanreiche Stoffstrom (e) Stickstoff enthält, wobei der in dem methanreichen Stoffstrom (e) enthaltene Stickstoff teilweise oder vollständig in den wasserstoffreichen Stoffstrom (f) überführt und in diesem dem Ammoniaksyntheseverfahren (40) unterworfen wird.

5. Prozess (100) nach Anspruch 4, bei dem der methanreiche Stoffstrom (e) bis zu 20 Molprozent, insbesondere von 5 bis 10 Molprozent, Stickstoff enthält.

6. Prozess (100) nach einem der Ansprüche 3 bis 5, bei dem der sauerstoffreiche Stoffstrom (e) Stickstoff enthält, wobei der in dem sauerstoffreichen Stoffstrom (e) enthaltene Stickstoff teilweise oder vollständig in den wasserstoffreichen Stoffstrom (f) überführt und in diesem den Ammoniaksyntheseverfahren (40) unterworfen wird.

7. Prozess (100) nach Anspruch 6, bei dem der sauerstoffreiche Stoffstrom (e) bis zu 20 Molprozent, insbesondere von 5 bis 10 Molprozent, Stickstoff enthält.

8. Prozess (100) nach einem der vorstehenden Ansprüche, bei dem aus dem Produktstrom (e) ferner ein oder mehrere olefinreiche Stoffströme (g, h) und unter Einbeziehung des Luftzerlegungsverfahrens (10) ein oder mehrere weitere sauerstoffreiche Stoffströme (n) gebildet werden, wobei der oder die olefinreichen Stoffströme (g, h) und der oder die weiteren sauerstoffreichen Stoffströme (n) einem Epoxidierungsverfahren (60) unterworfen werden.

9. Prozess (100) nach einem der vorstehenden Ansprüche, bei dem aus dem Produktstrom (e) wenigstens ein weiterer Stoffstrom gebildet wird, der erneut dem Verfahren zur oxidativen Methankopplung (20) unterworfen wird.

10. Prozess (100) nach einem der vorstehenden Ansprüche, bei dem Abwärme des Verfahrens zur oxidativen Methankopplung (20) zur Vorwärmung oder Erwärmung eines oder mehrerer Stoffströme und/oder eines oder mehrerer Reaktoren, die in dem Syntheseverfahren (40, 50) zur Erzeugung des oder der stickstoffhaltigen Syntheseprodukte eingesetzt werden, verwendet wird.

11. Anlagenverbund, der eine Luftzerlegungsanlage und wenigstens einen zur Durchführung eines Verfahrens zur oxidativen Methankopplung (20) eingerichteten Reaktor umfasst, wobei der Anlagenverbund Mittel aufweist, die dafür eingerichtet sind, unter Einbeziehung eines in der Luftzerlegungsanlage durchgeführten Luftzerlegungsverfahrens (10) einen sauerstoffreichen Stoffstrom (b) zu bilden und diesen mit einem methanreichen Stoffstrom (e) in dem wenigstens einen Reaktor einem Verfahren zur oxidativen Methankopplung (20) zu unterwerfen, **dadurch gekennzeichnet, dass** Mittel vorgesehen sind, die dafür eingerichtet sind, aus einem Produktstrom (e) des Verfahrens zur oxidativen Methankopplung (20) einen kohlendioxidreichen Stoffstrom (i) zu bilden und einem Harnstoffsyntheseverfahren (50) zu unterwerfen.

12. Anlagenverbund nach Anspruch 11, der zur Durchführung eines Verfahrens nach einem der Ansprüche 1 bis 10 eingerichtet ist.
